# EUROPÄISCHE PATENTSCHRIFT

(11) **EP 2 198 886 B1**
(45) Veröffentlichungstag und Bekanntmachung des Hinweises auf die Patenterteilung: **29.06.2011**
(21) Anmeldenummer: 10001518.9
(22) Anmeldetag: 21.12.2006
(51) Int. Cl.: A61K 41/00, A61L 2/08

(54) **Verfahren zur Inaktivierung von Pathogenen in Spenderblut, Blutplasma oder Erythrozytenkonzentraten in flexiblen Behältnissen unter Bewegung**
Method for inactivating pathogens in donor blood, blood plasma or erythrocyte concentrates in flexible containers in motion
Procédé de désactivation de pathogènes dans le sang du donneur, le plasma ou les concentrés d'érythrocytes dans des récipients souples en mouvement

(30) Priorität: 23.12.2005 DE 102005062634
(43) Veröffentlichungstag der Anmeldung: 23.06.2010
(62) Teilanmeldung aus: 06840887.1
(73) Patentinhaber: BLUTSPENDEDIENST DER LANDESVERBÄNDE DES DRK NIEDERSACHSEN, SACHSEN-ANHALT, THÜRINGEN, OLDENBURG UND BREMEN GGMBH, 31830 Springe (DE)
(72) Erfinder: Mohr, Harald, 30177 Hannover (DE)
(74) Vertreter: Schupfner, Georg

(56) Entgegenhaltungen:
- WO-A-01/54738
- WO-A-01/54739
- WO-A-89/09067
- WO-A-02/092806

## Beschreibung

Gegenstand der Erfindung ist ein Verfahren zur Inaktivierung von Pathogenen wie Bakterien und Viren in Spenderblut (Blut), Blutplasma (Plasma) und/oder Erythrozytenkonzentraten (EKs) durch photodynamische Behandlung und/oder Bestrahlung mit ultraviolettem Licht.

Es ist bekannt, dass die therapeutische Anwendung von Blut und Blutzubereitungen das Risiko birgt, dass die Empfänger mit Viren und Bakterien infiziert werden. Genannt seien z. B. die Viren Hepatitis-B (HBV) und Hepatitis-C (HCV) sowie die Aids-Erreger HIV-1 und HIV-2. Das Risiko besteht immer dann, wenn bei der Herstellung entsprechender Präparate kein Schritt zur Inaktivierung bzw. Eliminierung von Pathogenen Anwendung findet.

Es gab bzw. gibt eine Vielzahl von Bemühungen, Blutzubereitungen mittels photodynamischer Methoden zu dekontaminieren. Das Prinzip beruht darauf, das betreffende Produkt in Gegenwart einer photoaktiven Substanz (einem Photosensitizer) zu belichten. Das eingestrahlte Licht muss einen Wellenlängenbereich beinhalten, der vom Photosensitizer absorbiert und von dem er aktiviert werden kann. Die absorbierte Energie wird entweder direkt auf die betreffende Zielstruktur (z.B. die Nukleinsäure oder Oberflächenproteine eines Virus) transferiert, die dadurch zerstört wird, oder aber auf gelöste Sauerstoffmoleküle, die dadurch aktiviert werden. Es entsteht Singlet-Sauerstoff, der eine ausgeprägte viruzide und bakterizide Aktivität hat.

Idealerweise besitzt der verwendete Photosensitizer eine hohe Affinität für essenzielle Bestandteile von Viren und andere Pathogene, z.B. für deren Nukleinsäuren und nur eine geringe oder gar keine für die Bestandteile des Präparats, das dekontaminiert werden soll. Als Resultat der photodynamischen Behandlung werden in diesem Fall die Pathogene inaktiviert, während die Produktaktivität erhalten bleibt. Als ein geeigneter Photosensitizer für die Behandlung von Plasma ist z.B. der Phenothiazinfarbstoff Methylenblau beschrieben; für die Dekontamination von Thrombozytenkonzentraten wird Riboflavin (Vitamin B2) verwendet und zur Dekontamination von EKs sind Phthalocyanine erprobt. Allerdings kamen Verfahren zur photodynamischen Pathogeninaktivierung in EKs bisher nicht über den Labormaßstab hinaus.

Dies gilt noch mehr für Blut selbst. Ein Hauptgrund hierfür dürfte darin zu suchen sein, dass das eingestrahlte Licht eine bestimmte Intensität haben muss, um den verwendeten Photosensitizer aktivieren zu können und dass Blut und BKs nur eine sehr geringe Durchlässigkeit für Licht jeder Wellenlänge besitzen. Das Problem ist natürlich auch bei Plasma gegeben, wenn auch nicht im gleichen Ausmaß.

Es ist auch bekannt, dass man durch die bloße Bestrahlung mit kurzwelligem ultravioletten (UV-) Licht, d.h. im Wellenlängenbereich zwischen ca. 200 und 320 nm, insbesondere 200 bis kleiner 300 nm (UVB- und UVC) ebenfalls Pathogene inaktivieren kann. Oberhalb von 320 nm ist die Energie der Strahlung zu gering, um Mikroorganismen und Viren zu inaktivieren. Gegenüber chemischen, photochemischen und photodynamischen Methoden zur Pathogeninaktivierung besitzt die bloße Bestrahlung mit UV-Licht grundsätzlich den Vorteil, für sich allein wirksam zu sein und nicht des Zusatzes reaktiver Chemikalien oder photoaktiver Substanzen zu bedürfen.

Am effektivsten zur direkten Pathogeninaktivierung ist UVC. Es besitzt allerdings den Nachteil, dass es proteinhaltige Lösungen wie Plasma bzw. trübe Suspensionen (z.B. Blut und EKs) nur bis zu einer sehr geringen Eindringtiefe durchdringt. UVC wurde während des zweiten Weltkrieges und noch kurz danach eingesetzt, um Plasma und Albuminlösungen zu sterilisieren, vor allem um Hepatitis-Viren zu inaktivieren. Man ging damals so vor, dass die Lösung in einer Durchflussapparatur als dünner Film an einer UVC-Lichtquelle vorbei geleitet wurde. Die Methode erwies sich als nicht genügend sicher und wurde aufgegeben (Kallenbach NR, Cornelius PA, Negus D, et al. Inactivation of viruses by ultraviolet light. Curr. Stud. Hematol. Blood Transfus. 1989, 56, 70-82).

In heutiger Zeit werden weiterentwickelte nach dem gleichen Prinzip arbeitende Verfahren eingesetzt, um therapeutische Plasmaprotein-Präparationen zu sterilisieren. In allen Fällen ging bzw. geht es darum, größere Volumina zu behandeln; d.h. Plasma-Pools bzw. Proteinlösungen von bis zu einigen hundert Litern und sogar mehr (Hart H, Reid K, Hart W. Inactivation of viruses during ultraviolet light treatment of human intravenous immunoglobulin and albumin. Vox Sang 1993; 64(2):82-88. und Chin S, Williams B, Gottlieb P, et al. Virucidal short wavelength ultraviolet light treatment of plasma and factor VIII concentrate: protection of proteins by antioxidants; Blood 1995;86(11):4331-4336).

Zur Sterilisierung einer Vielzahl einzelner Einheiten von Blutspenden, Plasma oder von EKs, mit Volumina von allenfalls bis zu einigen Hundert ml - sind die genannten Durchflussapparaturen nicht geeignet. Gerade dies ist aber in der täglichen Praxis einer Blutbank vonnöten.

UVB ist gleichfalls mikrobiozid und viruzid, wenn auch nicht im gleichen Ausmaß wie UVC. Es penetriert proteinhaltige Lösungen und trübe Suspensionen etwas besser als UVC, allerdings lässt sich seine Eindringtiefe, z.B. in Plasma, auch nur im Bereich weniger Millimeter feststellen.

Plasma und EKs werden meist aus einzelnen Blutspenden isoliert, oder aber durch maschinelle Apherese von einzelnen Spendern gewonnen. Das Volumen der Präparate liegt im Allgemeinen zwischen ca. 200 und 350 ml. Das Volumen von Blutspenden liegt meist zwischen 450 und 500 ml. Die Präparate werden in flachen Kunststoffbeuteln im Allgemeinen entweder tiefgefroren (Plasma) oder bei ca. 4°C (Blutspenden, EKs) gelagert.

Wünschenswert wäre es, die genannten Präparate in derartigen Beuteln zu sterilisieren. Allerdings besteht hierbei das erwähnte Problem, dass sie fast undurchlässig für UV-Licht sind, Blut und EKs außerdem für sichtbares Licht.

Überraschenderweise wurde gefunden, dass obiges Problem durch ein Verfahren gemäß Anspruch 1 gelöst wird. Bevorzugte Ausführungsformen sind Gegenstand der abhängigen Ansprüche oder nachfolgend geschildert.

Nach der vorliegenden Erfindung werden die Präparate, d.h. Spenderblut (Blut), Blutplasma (Plasma) und/oder Erythrozytenkonzentrate (EKs) in ihren Belichtungsbeuteln in geeigneter Weise bewegt, so dass eine ständige Umwälzung der Proben im Behältnis erfolgt. Die Bewegung erfolgt hierbei so heftig, dass sich innerhalb der Flüssigkeit bzw. Suspension bereichsweise Schichten ausbilden, die so dünn sind, dass sie vom eingesetzten Licht durchdrungen werden können. Die Bewegung muss gleichzeitig so sein, dass die Flüssigkeit bzw. Suspension im Beutel wirksam vermischt wird. Beides wird realisiert wenn folgende Voraussetzungen gegeben sind:
1. Die Belichtungsbeutel sind hochflexibel, und sie werden während der Belichtung nicht fixiert, z.B. nicht zwischen Glas- oder Quarzplatten eingeklemmt. Sie passen sich somit der Gestaltsänderung des Plasmas bzw. der Suspension (Blut, EK) an, die sich ergibt, wenn die Beutel bewegt werden.
2. Die Belichtungsbeutel sind zu maximal 30 % , insbesondere zu maximal 15%, des maximalen Füllvolumens gefüllt.
3. die Beutel werden heftig bewegt, z.B. entweder horizontal (linear in Hin- und Her-Richtung bzw. kreis- oder ellipsenfömig) oder aber vertikal (gewippt).

Unter heftiger Bewegung ist hierbei (einzeln oder gemeinsam) Folgendes zu verstehen:
1. sie geht über eine bloße Bewegung hinaus, durch die bei Flüssigkeiten bzw. Suspensionen eine Vermischung bewirkt wird.
2. Innerhalb der Flüssigkeiten bzw. Suspensionen, die heftig bewegt werden, bilden sich zumindest zeitweise, auch an verschiedenen Stellen, Bereiche aus, die so dünn sind, dass sie von UV- bzw. sichtbarem Licht (letzteres gilt für trübe bzw. gefärbte Flüssigkeiten bzw. Suspensionen, z.B. EKs) durchdrungen werden können.
3. Die Umkehrung der Bewegungsrichtung ist bei heftiger Bewegung so abrupt, dass der größere Teil des Präparats, das sich im Belichtungsbeutel befindet, sich infolge seiner Trägheit weiter in die ursprüngliche Richtung bewegt und somit der zurückbleibende Rest eine dünne Schicht ausbilden kann, die für das eingesetzte Licht durchdringbar ist.

In Verbindung mit der ständigen Durchmischung, die gleichzeitig stattfindet, wird letztendlich das gesamte Präparat (und die darin enthaltenen Pathogene) belichtet, und es wird somit sterilisiert.

Der Belichtungsbeutel ist im liegenden gefüllten Zustand nur einige mm dick, z.B. kleiner 10 mm, vorzugsweise kleiner als 5 mm und ist bestimmt, Probenvolumina von z.B. 200 bis 500 ml aufnehmen. Das maximale Fassungsvermögen (Volumen) des Belichtungsbeutels ist aber um mindestens Faktor 3, i.d.R. um mindestens 6,66 mal größer vorzugsweise mindestens 10 oder sogar mindestens 20 mal größer als das tatsächliche in ihm enthaltene zu behandelnde Probenvolumen.

Nach einer Ausgestaltung der Erfindung erfolgt die Bewegung, insbesondere die Amplitude der Bewegung so, dass sich innerhalb der Präparate Bereiche ausbilden, in der die Schichtdicke regelmäßig zeitweise geringer als 0,05 mm ist.

Die Belichtungsbeutel haben insbesondere ein Volumen von bis zu 5000 ml.

Die Belichtungsbeutel werden z.B. durch Schütteln, Wippen und/oder durch Rotation bewegt. Die Belichtungsbeutel werden bevorzugt während zumindest dreiviertel, insbesondere zumindest fünf/sechstel der gesamten Belichtungsdauer bewegt. Das Bewegen bzw. das Schütteln kann durch einen Orbitalschüttler, Plattformschüttler, Wippschüttler oder Taumelschüttler erfolgen.

Nach einer Ausgestaltung werden die Belichtungsbeutel während der Belichtung z.B. ständig mit einer Amplitude von größer 0,2 mm, vorzugsweise größer 1 cm und insbesondere 1 bis 15 cm oder 2 bis 8 cm zumindest in der x-Richtung und ggf. auch in der y-Richtung (y-Richtung rechtwinkelig zur x-Richtung) bewegt. Unabhängig hiervon beträgt die Frequenz der Richtungsänderung der Bewegung größer 0,5 Hz, vorzugsweise 1 bis 10 Hz.

Nach einer weiteren Ausgestaltung der Erfindung stammen die Präparate aus einer Einheit von einem bis 6 Spendern, vorzugsweise einem Spender.

Im Falle einer photodynamischen Behandlung in Gegenwart einer photoaktiven Substanz erfolgt die Bestrahlung vorzugsweise mit Wellenlängen im Absorptionsbereich (+/- 20 nm) des bzw. der eingesetzten Photosensitizer(s).

### Experimentelle Untersuchungen

Die beschriebenen Versuche illustrieren die Wirksamkeit des Verfahrens und sind nicht auf die Inaktivierung der genannten Viren beschränkt. Es besteht auch keine Einschränkung auf die bei den beschriebenen Versuchen eingesetzten Plasmen bzw. EKs, die aus Blutspenden stammten; das erfindungsgemäße Verfahren ist auch bei Präparaten anwendbar, die auf andere Weise hergestellt werden. Alle beschriebenen Versuche wurden drei bis sechs Mal durchgeführt. Die angegebenen Ergebnisse stellen jeweils die Mittelwerte aus diesen Versuchen dar.

### Plasma-Einheiten und Erythrozytenkonzentrate

Die eingesetzten Plasma-Einheiten und die EKs wurden aus einzelnen Blutspenden mittels üblicher Verfahren hergestellt. Sie hatten ein Volumen von ca. 250 bis 300 bzw. bis zu 350 ml und wurden in üblichen Kunststoffbeuteln für Blutpräparate aufbewahrt. Restliche Leukozyten bzw. Thrombozyten wurden durch Filtration entfernt. Die EKs waren im Stabilisatormedium SAG-M suspendiert. Die Plasmen wurden bei Temperaturen unter -30°C gelagert und für die Versuche im Wasserbad aufgetaut. Die EKs wurden bei 4 bis 8°C im Kühlraum gelagert.

### Virologische Untersuchungen

Plasma- bzw. EK-Aliquots wurden mit Vesicular Stomatitisvirus (VSV, Stamm Indiana, ATCC VR 158) bzw. Sinbis-Virus (ATCC VR-68) bzw. Suid Herpesvirus (SHV-1, Pseudorabiesvirus, Stamm Aujeszky, ATCC VR-135) gespikt. Virustiter wurden mittels CPE-Assay (CPE = cytopathischer Effekt) bestimmt. Sie werden als TCID₅₀ (TCID = Tissue culture infective dose) angegeben. Als Indikatorzellen dienten Vero-Zellen. Die initiale Viruskonzentration bei den durchgeführten Versuchen betrug ca. 10⁵ bis 10⁷ TCID₅₀.

### Belichtungsanlagen, Belichtungsbeutel

Eine der verwendeten Belichtungsanlagen war mit Röhren ausgestattet, die UVC-Licht (Wellenlänge: 254 nm) emittierten. Die Bestrahlung erfolgte von beiden Seiten der aufgelegten Belichtungsbeutel, d.h. von oben und von unten. Eine zweite Belichtungsanlage war mit Röhren ausgestattet, die UVB-Licht (280-320 nm) emittierten. Die Bestrahlung erfolgte ebenfalls von beiden Seiten. Eine dritte Belichtungsanlage war mit LEDs (Licht emittierende Dioden) ausgestattet, die intensives rotes Licht im Wellenlängenbereich um 635 nm emittierten. Alle drei Anlagen wurden im Betrieb auf einem Orbitalschüttler platziert (Hersteller Firma Bühler, Tübingen; Typ SM 25) der bis zu 100 Umdrehungen pro Minute durchführte. Die verwendeten Belichtungsbeutel bestanden aus dünner UVdurchlässiger und hochflexibler Kunststofffolie.

### Versuchsbeispiel 1

### Inaktivierung von VSV in Plasma durch UVC: Einfluss der Schüttelgeschwindigkeit und der freien Beweglichkeit des Plasmas während der Bestrahlung

Plasma-Einheiten in Belichtungsbeuteln wurden mit VSV gespikt und für 2 min mit UVC bestrahlt. Eine Probe wurde mit 100 rpm geschüttelt und war während der Bestrahlung fest zwischen zwei Quarzplatten eingespannt. Die anderen Proben lagen lediglich auf einer Quarzplatte auf, so dass sie sich während des Schüttelns innerhalb des Beutels bewegen konnten. Die Drehzahl des Schüttlers wurde zwischen 30 und 100 rpm variiert. Die Ergebnisse sind in der Tab. 1 zusammengestellt. In der fest eingespannten Probe wurde der Virustiter lediglich um einen Faktor von ca. 0,3 log₁₀ reduziert.

**Tabelle 1**

| **Schüttelfre quenz (rpm)** | **Virustiter (log₁₀TCID₅₀)** | **Bemerkung** |
|---|---|---|
| 0 | 6,21±0,69 | Kontrolle |
| 30 | 5,78±0,27 | lose aufgelegt |
| 50 | 4,59±0,04 | lose aufgelegt |
| 75 | 0,92±0,24 | lose aufgelegt |
| 100 | 0,35±0,52 | lose aufgelegt |
| 100 | 5,92±0,11 | eingespannt |

Bei den lose platzierten Proben hatte die Drehzahl einen unmittelbaren Einfluss auf das Ausmaß der Virusinaktivierung: während bei 30 bzw. 50 rpm die Inaktivierungsfaktoren im Vergleich zu den unbehandelten Kontrollproben nur ca. 1,1 bzw. 2,4 log₁₀ betrugen, stiegen sie bei 75 rpm auf ca. 5,1 und bei 100 rpm auf ca. 6,6 log₁₀ an. Die Ergebnisse dieses Versuchs belegen, dass Plasma während der Behandlung intensiv geschüttelt werden muss, damit die Bestrahlung mit UV-Licht wirksam werden kann. Damit sich aber der Schütteleffekt auch auswirkt, müssen die Proben lose platziert sein, damit sich während des Schüttelns dünne Schichten ausbilden, die durchstrahlt werden können.

### Versuchsbeispiel 2

### Inaktivierung von VSV, Sinbis-Viren und von SHV-1 in Plasma durch die Bestrahlung mit UVC: Inaktivierungskinetiken

Plasma-Einheiten wurden mit VSV, Sindbis-Viren bzw. SHV-1 gespikt und für 1-5 min bestrahlt. Die auf dem Orbitalschüttler lose platzierten Proben wurden mit 100 rpm bewegt. Kontrollproben wurden für 5 min bestrahlt, aber nicht geschüttelt. Die Ergebnisse der Versuche sind in der Tab. 2 zusammengestellt. In den geschüttelten Proben wurde der Titer von VSV innerhalb von 3 min um einen Faktor von mehr als 6,5 log₁₀ abgereichert, während der Inaktivierungsfaktor in der nicht geschüttelten Kontrollprobe 1,5 log₁₀ nicht überstieg. Sindbis-Viren erwiesen sich als stabiler als VSV; aber der große Unterschied zwischen geschüttelten und ungeschüttelten Proben zeigte sich auch hier: Nach einer Bestrahlungszeit von 5 min hatte sich der Virustiter in der geschüttelten Probe um ca. 5.1 log₁₀ verringert, in der ungeschüttelten dagegen nur um 0,30 log₁₀. Ein ähnliches

**Tabelle 2**

| | | **Virustiter (log₁₀TCID₅₀)** | | |
|---|---|---|---|---|
| **UVC (min)** | **Schütteln** | **VSV** | **Sinbis** | **SHV-1** |
| Kontrolle | - | 6,74±0,32 | 7,01±0,24 | 4,95±0,23 |
| 2 | + | 0,95±0,31 | 4,68±0,21 | 2,56±0,25 |
| 3 | + | ≤0,24±0,00 | 3,27±0,16 | 1,67±0,37 |
| 4 | + | ≤0,24±0,00 | 2,10±0,12 | 0,66±0,29 |
| 5 | + | ≤0,24±0,00 | 1,86±0,09 | 0,42±0,21 |
| 5 | - | 5,69±0,18 | 6,73±0,16 | 4,65±0,16 |

Bild ergab sich, wenn SHV-1 eingesetzt würde: in den geschüttelten Proben verminderte sich der Virustiter innerhalb von 4 bis 5 min um einen Faktor von 4,3 bis 4,5 log₁₀; in der nicht geschüttelten Probe war er nach 5 min Bestrahlung nur um 0,3 log₁₀ reduziert worden.

### Versuchsbeispiel 3 Inaktivierung von VSV in Plasma durch die Bestrahlung mit UVB: Inaktivierungskinetik

Plasma-Einheiten wurden mit VSV gespikt und für 1 bis 5 min bestrahlt. Die auf dem Orbitalschüttler lose platzierten Proben wurden mit 100 rpm bewegt. Eine Kontrollprobe wurde für 5 min bestrahlt, aber nicht geschüttelt. Wie aus der Tab. 3 hervor geht, wurde in den geschüttelten Proben der Virustiter innerhalb von 5 min um einen Faktor von 6,36 log₁₀ abgereichert, in der nicht geschüttelten Kontrollprobe dagegen nur um ca. 1,5 log₁₀. Die Ergebnisse zeigen, dass das entdeckte Phänomen - die Steigerung der Pathogeninaktivierung in lose platzierten Proben durch intensives Schütteln - nicht auf UVC beschränkt ist.

**Tabelle 3**

| **UVB (min)** | **Schütteln** | **Virustiter (log₁₀ TCID₅₀)** |
|---|---|---|
| Kontrolle | - | 7,00±016 |
| 2 | + | 4,70±0,08 |
| 3 | + | 3,68±0,12 |
| 4 | + | 2,23±0,23 |
| 5 | + | 0,64±0,08 |
| 5 | - | 5,52±0,08 |

### Versuchsbeispiel 4

### Inaktivierung von VSV in Plasma durch photodynamische Behandlung mit Methylenblau und Licht.

Plasma-Einheiten wurden mit VSV gespikt, mit 0,25 µmol/l des Photosensitizers Methylenblau (MB) versetzt und auf dem Orbitalschüttler bei einer Drehzahl von 100 rpm für bis zu 30 min mit rotem LED-Licht bestrahlt. Kontrollproben wurden in Gegenwart der gleichen Konzentration von MB für 20 min belichtet, aber währenddessen nicht bewegt.

Wie die Tab. 4 zeigt, war das Ausmaß der Virusinaktiverung in den geschüttelten viel größer als in den nicht geschüttelten Proben. In den ersteren hatte sich der Virustiter nach 20 min um einen Faktor von ca. 4,4 log₁₀ vermindert; nach 30 min um ca. 5,8 log₁₀. In den unbewegten Proben war der Reduktionsfaktor nach 20 min dagegen nicht höher als ca. 2,7 log₁₀.

**Tabelle 4**

| **MB/Licht (min)** | **Schütteln** | **Virustiter (Log₁₀TCID₅₀)** |
|---|---|---|
| Kontrolle | - | 6,72±0,24 |
| 10 | + | 4,95±0,68 |
| 20 | + | 2,30±0,88 |
| 30 | + | 0,94±0,87 |
| 20 | - | 4,04±0,54 |

### Versuchsbeispiel 5

### Inaktivierung von VSV in EKs durch photodynamische Behandlung mit Methylenblau und Licht.

EK-Aliquots wurden mit VSV gespikt, mit 5 µmol/l des Photosensitizers Methylenblau (MB) versetzt und auf dem Orbitalschüttler bei einer Drehzahl von 100 rpm für bis zu 30 min mit rotem LED-Licht bestrahlt. Kontrollproben wurden dagegen während der Belichtung nicht bewegt. Das eindeutige Ergebnis des Versuchs zeigt die Tab. 5. Es ist offensichtlich, dass die Virusinaktiverung in den geschüttelten bedeutend schneller als in den ungeschüttelten EK-Proben ablief. In den Proben, die während der Behandlung bewegt wurden, war das Virus nach 30 min fast völlig komplett inaktivert worden (Inaktivierungsfaktor 6,7 log₁₀). Der Reduktionsfaktor in den unbewegten Proben betrug nach 30 min dagegen nur ca. 2,7 log₁₀.

Die Ergebnisse der Versuchsbeispiele 4 und 5 belegen, dass auch die Wirksamkeit der photodynamischen Behandlung von Plasma oder von Erythrozytenkonzentraten enorm gesteigert wird, wenn die Proben während der Belichtung stark geschüttelt werden.

**Tabelle 5**

| **MB/Licht (min)** | **Schütteln** | **Virustiter (Log₁₀TCID₅₀)** |
|---|---|---|
| Kontrolle | - | 7,04±0,26 |
| 10 | + | 2,62±0,31 |
| 20 | + | 0,89±0,21 |
| 30 | + | 0,30±0,12 |
| 10 | - | 5,07±0,26 |
| 20 | - | 5,25±0,31 |
| 30 | - | 4,35±0,27 |

## Patentansprüche

1. Verfahren zur Inaktivierung von Pathogenen in Spenderblut, Blutplasma und/oder Erythrozytenkonzentraten, umfassend die folgenden Schritte:
• Bereitstellen der Blutspenden bzw. der aus Spenderblut und/oder durch maschinelle Apherese gewonnenen Präparate,
(a) Zufügen einer geeigneten photoaktiven Substanz und photodynamische Behandlung durch Bestrahlen mit Licht umfassend oder ausschließlich bestehend aus Wellenlängen im Absorptionsbereich der photoaktiven Substanz, wobei die photoaktive Substanz ein oder mehrere Phenothiazin-Farbstoffe sind, eine oder mehrere Phthalocyanin-Verbindungen und/oder eine oder mehrere Porphyrin-Verbindungen sind,
oder
(b) Aussetzen der Präparate einer Bestrahlung mit ultraviolettem (UV-) Licht bei Wellenlängen von 200 bis 320 nm, wobei die Bestrahlung mit UVB von kleiner 320 nm bis 280 nm und einer Lichtenergie von 0,3 bis 10 J/cm² und/oder UVC von kleiner 280 nm bis 200 nm erfolgt,
• wobei die Präparate aus einer Vielzahl von einzeln handhabbaren und getrennt aufbewahrten Einheiten bestehen und
• die Präparate sich jeweils in flexiblen Licht- (Alternative (a)) bzw. UVdurchlässigen (Alternative (b)) flächigen Belichtungsbeuteln befinden,
**dadurch gekennzeichnet, dass**
• die Belichtungsbeutel zu weniger als 30 Vol% des maximalen Füllvolumens der Belichtungsbeutel gefüllt sind und
• die Belichtungsbeutel während der photodynamischen Behandlung und/oder der Bestrahlung mit UV-Licht so bewegt werden, dass der Inhalt des Belichtungsbeutels umgewälzt wird und sich durch die Bewegung Zonen wechselnder Schichtdicken ausbilden.

2. Verfahren gemäß Anspruch 1 **dadurch gekennzeichnet, dass** die Pathogene Viren und / oder Bakterien sind.

3. Verfahren gemäß zumindest einem der vorhergehenden Ansprüche, **dadurch gekennzeichnet, dass** durch die Bewegung die Zonen Bereiche aufweisen, für die sich regelmäßig zeitweise Schichtdicken unter 1 mm, vorzugsweise unter 0,05 mm ergeben.

4. Verfahren gemäß zumindest einem der vorhergehenden Ansprüche, **dadurch gekennzeichnet, dass** die Summe der Flächen der Unterseite und der Oberseite der Belichtungsbeutel, die mit dem Beutelinhalt in Kontakt steht bzw, stehen kann, mehr als 90 Flächenprozent, vorzugsweise mehr als 99 Flächenprozent der inneren Gesamtoberfläche des Beutelinhalts ausmacht.

5. Verfahren gemäß zumindest einem der vorhergehenden Ansprüche, **dadurch gekennzeichnet, dass** im Falle der Ausführungsform (b) die Bestrahlung UVB und/oder UVC, insbesondere UVC von kleiner 260 nm bis 220 nm, ist oder umfasst und vorzugsweise ausschließlich aus Bestrahlung mit Wellenlängen der genannten Bereiche besteht.

6. Verfahren gemäß zumindest einem der vorhergehenden Ansprüche, **dadurch gekennzeichnet, dass** die Bestrahlung mit UVB mit einer Lichtenergie von 0.5 bis 5 J/cm² erfolgt.

7. Verfahren gemäß zumindest einem der Ansprüche 1 bis 5, **dadurch gekennzeichnet, dass** die Bestrahlung mit UVC mit einer Lichtenergie von 0.01 bis 5 J/cm², insbesondere 0,1 bis 2 J/cm² erfolgt.

8. Verfahren gemäß zumindest einem der vorhergehenden Ansprüche, **dadurch gekennzeichnet, dass** die Belichtungsbeutel in der Apparatur, in der sie bewegt und bestrahlt werden, beweglich gehaltert sind und insbesondere nicht zwischen zwei Flächen eingespannt sind.

9. Verfahren gemäß zumindest einem der vorhergehenden Ansprüche, **dadurch gekennzeichnet, dass** die Präparate Plasma sind und zu mehr als 80 Gew.% aus Blutplasma bestehen.

10. Verfahren gemäß zumindest einem der Ansprüche 1 bis 8, wobei die Präparate EK-Präparate sind und einen Hämatokrit zwischen 10 und 75 Gew.%, vorzugsweise zwischen 20 bis 60 Gew.% aufweisen.

11. Verfahren gemäß zumindest einem der vorhergehenden Ansprüche, **dadurch gekennzeichnet, dass** die photoaktive Substanz ein oder mehrere Phenothiazin-Farbstoffe sind, nämlich Thionin, Methylenblau, Toluidinblau und/oder die Azurfarbstoffe A, B und/oder C.

12. Verfahren gemäß zumindest einem der vorhergehenden Ansprüche, **dadurch gekennzeichnet, dass** die Belichtungsbeutel zu maximal 15% des maximalen Füllvolumens gefüllt sind.

13. Verfahren gemäß zumindest einem der vorhergehenden Ansprüche, **dadurch gekennzeichnet, dass** Blutplasma gemäß Schritt (a) und in Abwesenheit eines Photosensitizer(s) behandelt wird.

14. Verfahren gemäß zumindest einem der vorhergehenden Ansprüche, **dadurch gekennzeichnet, dass** die Belichtungsbeutel einseitig aufgelegt sind, so dass während und durch das Bewegen bzw. Schütteln sich die Höhe der Belichtungsbeutel, bezogen auf die Distanz (Flächennormale) zwischen Fläche, auf der die Belichtungsbeutel aufliegen und Schnittpunkt mit der oberen Fläche des Belichtungsbeutels über die gesamte obere Fläche des Belichtungsbeutels betrachtet, ständig ändert.

15. Verfahren gemäß zumindest einem der vorhergehenden Ansprüche, **dadurch gekennzeichnet, dass** der Belichtungsbeutel eine mittlere Füllhöhe von weniger als 5 mm aufweist und durch die Bewegung ständig Wellentäler erzeugt werden, die Schichtdicken von kleiner der Hälfte der mittleren Füllhöhe, vorzugsweise Schichtdicken von kleiner 1 mm oder sogar kleiner 0,05 mm, aufweisen.

## Claims

1. Method for inactivating pathogens in donor blood, blood plasma and/or erythrocyte concentrates, comprising the following steps:
- providing the blood donations and/or the preparations obtained from donor blood and/or by machine apheresis,
- (a) adding a suitable photoactive substance and photodynamic treatment by irradiation with light, comprising or consisting exclusively of wavelengths in the absorption range of the photoactive substance, wherein the photoactive substance are one or more phenothiazine dyes, one or more phtalocyanine compounds and/or one or more porphyrin compounds,
or
- (b) subjecting the preparations an irradiation with ultraviolet (UV) light at wavelengths of 200 nm to 320 nm, wherein the irradiation is applied with UVB of less than 320 to 280 nm and a light energy of 0.3 to 10 J/cm² and/or with UVC of less than 280 nm to 200 nm,
- wherein the preparations consist of numerous units that can be handled individually and stored separately, and
- the preparations are in flat, flexible light-permeable (alternative (a)) and/or UV-permeable (alternative (b)) irradiation bags,
**characterized in that**
- the irradiation bags are filled to less than 30 vol% of the maximum filling volume of the irradiation bags, and
- the irradiation bags are agitated during the photodynamic treatment and/or the irradiation with UV light, so that the contents of the irradiation bags are circulated and zones of variable layer thickness develop due to this movement.

2. The method according to Claim 1, **characterized in that** the pathogens are viruses and/or bacteria.

3. The method according to at least one of the preceding claims, **characterized in that** due to the movement, the zones have regions which regularly have layer thicknesses of temporarily less than 1 mm, preferably less than 0.05 mm.

4. The method according to at least one of the preceding claims, **characterized in that** the sum of the areas of the bottom side and the top side of the irradiation bags that are/can be in contact with the contents of the bag amounts to more than 90% by area, preferably more than 99% by area, of the total internal surface area of the bag contents.

5. The method according to at least one of the preceding claims, **characterized in that** in the case of embodiment (b), the irradiation is or includes UVB and/or UVC of less than 260 nm to 220 nm and preferably consists exclusively of radiation with wavelengths in the ranges given above.

6. The method according to at least one of the preceding claims, **characterized in that** the irradiation is applied with UVB having a light energy 0.5 to 5 J/cm².

7. The method according to at least one of Claims 1 through 5, **characterized in that** the irradiation is applied with UVC having a light energy of 0.01 to 5 J/cm², in particular 0.1 to 2J/cm²_{.}

8. The method according to at least one of the preceding claims, **characterized in that** the irradiation bags are held movably in the apparatus in which they are moved and irradiated, and in particular they are not clamped between two surfaces.

9. The method according to at least one of the preceding claims, **characterized in that** the preparations are plasma and consist of more than 80% by weight blood plasma.

10. The method according to at least one of Claims 1 through 8, wherein the preparations are erythrocyte preparations and have a hematocrit between 10% and 75% by weight, preferably between 20% and 60% by weight.

11. The method according to at least one of the preceding claims, **characterized in that** the photoactive substances include one or more phenothiazine dyes, namely thionine, methylene blue, toluidine blue and/or the azure dyes A, B and/or C.

12. The method according to at least one of the preceding claims, **characterized in that** the irradiation bags are filled to max. 15% of the maximum filling volume.

13. The method according to at least one of the preceding claims, **characterized in that** blood plasma is treated according to step (a) and in the absence of a photosensitizer.

14. The method according to at least one of the preceding claims, **characterized in that** the irradiation bags are placed on one side, so that the height of the irradiation bags, based on the distance (surface normal) between the surface on which the irradiation bags are lying and the point of intersection with the upper surface of the irradiation bag, changes constantly during and due to the movement, or shaking, when viewed over the total upper surface of the irradiation bag.

15. The method according to at least one of the preceding claims, **characterized in that** the irradiation bag has an average filling height of less than 5 mm and wave valleys having layer thicknesses of less than half the average filling height, preferably layer thicknesses of less than 1 mm or even less than 0.05 mm are produced constantly due to the movement.

## Revendications

1. Procédé pour inactiver des pathogènes dans du sang de donneurs, du plasma sanguin et/ou des concentrés érythrocytaires, comprenant les étapes suivantes consistant à :
- mettre à disposition des dons de sang ou des préparations obtenues à partir de sang de donneurs et/ou par aphérèse automatique,
(a) ajouter une substance photoactive appropriée et procéder au traitement photodynamique par irradiation avec de la lumière, comprenant ou se composant exclusivement de longueurs d'ondes situées dans la plage d'absorption de la substance photoactive, où la substance photoactive est un ou plusieurs colorants à la phénothiazine, un ou plusieurs composés de phtalocyanine et/ou un ou plusieurs composés de porphyrine,
ou
(b) soumettre les préparations à une irradiation avec une lumière ultraviolette (UV) à des longueurs d'ondes allant de 200 à 320 nm, où l'irradiation se fait avec des UVB de moins de 320 nm à 280 nm et à une énergie lumineuse de 0,3 à 10 J/cm² et/ou avec des UVC de moins de 280 nm à 200 nm,
- les préparations se composant d'une multitude d'unités qui peuvent être manipulées individuellement et sont conservées séparément, et
- les préparations se trouvant respectivement dans des poches d'irradiation planes et flexibles, perméables à la lumière (variante (a)) ou aux UV (variante (b)),
**caractérisé en ce que**
- les poches d'irradiation sont remplies à moins de 30 % du volume maximal de remplissage des poches d'irradiation et
- les poches d'irradiation sont agitées pendant le traitement photodynamique et/ou l'irradiation avec la lumière UV de sorte que le contenu des poches d'irradiation soit bien mélangé et que des zones d'épaisseurs de couches changeantes se forment par le biais de ce mouvement.

2. Procédé selon la revendication 1, **caractérisé en ce que** les pathogènes sont des virus et/ou des bactéries.

3. Procédé selon au moins une des revendications précédentes, **caractérisé en ce que**, du fait du mouvement, les zones présentent des sections pour lesquelles il résulte régulièrement des épaisseurs de couches temporaires inférieures à 1 mm, de préférence inférieures à 0,05 mm.

4. Procédé selon au moins une des revendications précédentes, **caractérisé en ce que** la somme des surfaces de la face inférieure et de la face supérieure des poches d'irradiation, qui est en contact ou peut être en contact avec le contenu de la poche, constitue plus de 90 % surfacique, de préférence plus de 99 % surfacique de la surface interne totale du contenu de la poche.

5. Procédé selon au moins une des revendications précédentes, **caractérisé en ce que**, dans le cas du mode de réalisation (b), l'irradiation avec les UVB et/ou avec les UVC, en particulier avec les UVC, est de moins de 260 nm à 220 nm, ou est comprise dans cette plage, et se compose de préférence exclusivement de l'irradiation aux longueurs d'ondes des plages susmentionnées.

6. Procédé selon au moins une des revendications précédentes, **caractérisé en ce que** l'irradiation avec les UVB s'effectue à une énergie lumineuse de 0,5 à 5 J/cm².

7. Procédé selon au moins une des revendications 1 à 5, **caractérisé en ce que** l'irradiation avec les UVC s'effectue à une énergie lumineuse de 0,01 à 5 J/cm², en particulier de 0,1 à 2 J/cm².

8. Procédé selon au moins une des revendications précédentes, **caractérisé en ce que** les poches d'irradiation sont maintenues par un support de manière à rester mobiles dans l'appareil, dans lequel elles sont agitées et irradiées, et ne sont notamment pas compressées entre deux surfaces.

9. Procédé selon au moins une des revendications précédentes, **caractérisé en ce que** les préparations sont du plasma et se composent à plus de 80 % en poids de plasma sanguin.

10. Procédé selon au moins une des revendications 1 à 8, dans lequel les préparations sont des préparations de concentré érythrocytaire et présentent un taux d'hématocrite situé entre 10 et 75 % en poids, de préférence entre 20 et 60 % en poids.

11. Procédé selon au moins une des revendications précédentes, **caractérisé en ce que** la substance photoactive est un ou plusieurs colorants à la phénothiazine, en particulier la thionine, le bleu de méthylène, le bleu de toluidine et/ou les colorants azur A, B et/ou C.

12. Procédé selon au moins une des revendications précédentes, **caractérisé en ce que** les poches d'irradiation sont remplies à au maximum 15 % du volume de remplissage maximal.

13. Procédé selon au moins une des revendications précédentes, **caractérisé en ce que** le plasma sanguin est traité selon l'étape (a) et en l'absence d'un photosensibilisant.

14. Procédé selon au moins une des revendications précédentes, **caractérisé en ce que** les poches d'irradiation sont posées sur une face de manière à ce que la hauteur des poches d'irradiation, par rapport à la distance (normale à la surface) entre la surface sur laquelle reposent les poches d'irradiation et le point d'intersection avec la surface supérieure de la poche d'irradiation, considéré sur toute la surface supérieure de la poche d'irradiation, se modifie constamment pendant et du fait du mouvement ou de l'agitation.

15. Procédé selon au moins une des revendications précédentes, **caractérisé en ce que** la poche d'irradiation présente une hauteur de remplissage moyenne de moins de 5 mm et que le mouvement provoque constamment des creux de vague qui présentent des épaisseurs de couches inférieures à la moitié de la hauteur de remplissage moyenne, de préférence des épaisseurs de couches de moins de 1 mm, voire même de moins de 0,05 mm.
